# FASCICULE DE BREVET EUROPEEN

(11) **EP 0 907 353 B1**
(45) Date de publication et mention de la délivrance du brevet: **11.02.2004**
(21) Numéro de dépôt: 97952099.6
(22) Date de dépôt: 18.12.1997
(51) Int. Cl.: A61K 7/48

(54) **COMPOSITION COSMETIQUE AMINCISSANTE INCLUANT UN EXTRAIT DE CHRYSANTHELLUM INDICUM**
CHRYSANTHELLUM INDICUM EXTRAKT ENTHALTENDES, KOSMETISCHES SCHLANKHEITSMITTEL
SLIMMING COSMETIC COMPOSITION CONTAINING A CHRYSANTHELLUM INDICUM EXTRACT

(30) Priorité: 09.01.1997 FR 9700323
(43) Date de publication de la demande: 14.04.1999
(73) Titulaire: LANATECH LABORATOIRE NATURE ET TECHNIQUE, 75116 Paris (FR)
(72) Inventeur: VACHER, Anne-Marie, F-78150 Le Chesnay (FR); FRITSCH, Marie-Claire, F-75007 Paris (FR)
(74) Mandataire: de Saint-Palais, Arnaud Marie
(86) Numéro de dépôt international: PCT/FR1997/002344
(87) Numéro de publication internationale: WO 1998/030200

(56) Documents cités:
- EP-A- 0 317 453
- EP-A- 0 493 151
- WO-A-98/20851
- FR-A- 2 233 071
- FR-A- 2 276 060
- FR-A- 2 456 116
- FR-A- 2 618 071
- US-A- 4 842 859
- DATABASE WPI Section Ch, Week 9739 Derwent Publications Ltd., London, GB; Class B04, AN 97-416092 XP002044018 & CN 1 113 691 A (ZHANG Z) , 27 décembre 1995
- BRASSEUR, T.: "MEDICAMENTS RENFERMANT DES FLAVONOIDES" JOURNAL DE PHARMACIE DE BELGIQUE, vol. 44, no. 6, 1989, pages 403-410, XP002044569
- HONORE-THOREZ D.: 'Description, identification et usages thérapeutiques de Chrysanthellum' J. PHARM. BELG. vol. 40, no. 5, 1985, pages 323 - 331

## Description

La présente invention concerne une composition cosmétique amincissante administrable par voie topique externe utilisable notamment, mais non exclusivement, pour le traitement préventif et/ou curatif de la cellulite.

D'une façon générale, on sait que chez la femme, le tissu gras situé dans l'hypoderme représente 15 à 20 % du poids corporel. Il est réparti de façon hétérogène au niveau des hanches, des fesses et de l'abdomen notamment.

L'hypoderme est hydrolipidique : 85 % de corps gras et 15 % d'eau (dont les 2/3 sont extracellulaires). Ce "coussin graisseux" est constitué de cellules lipidiques, les adipocytes. Grosses cellules vacuolisées presque entièrement remplies de triglycérides, les adipocytes sont regroupés en lobules délimités par des travées conjonctivovasculaires.

Chez la femme, ces cloisons sont verticales et perpendiculaires à la surface cutanée. En revanche chez l'homme, elles se disposent de façon oblique. Cette différence explique que la "peau d'orange" soit une manifestation typiquement féminine.

La partie conjonctive comporte des fibres de collagène et de réticuline et des cellules réticulo-endothéliales. La vascularisation est assurée par une artère et deux veines pour chaque lobule et par de nombreux capillaires qui parcourent les lobules et enserrent chaque adipocyte.

Ce rapprochement est primordial pour le transfert des lipides de l'adipocyte dans la circulation générale et inversement.

A cette vascularisation importante, s'ajoute un riche réseau lymphatique et une innervation qui chemine dans l'hypoderme avant d'aboutir dans le derme sus-jacent.

Les adipocytes, cellules essentielles du tissu gras, représentent une réserve énergétique majeure.

Les réserves lipidiques du tissu adipeux se renouvellent en permanence, signe d'un métabolisme cellulaire particulièrement actif, avec pour conséquence un "turn-over" rapide des lipides.

Ce métabolisme comporte trois phases : la lipogénèse (biosynthèse des acides gras), le stockage des lipides sous forme de triglycérides et la lipolyse (hydrolyse des triglycérides).

Il s'avère que pour différentes raisons (réaction vis-à-vis de composés toxiques, déséquilibre entre l'anabolisme protéique et lipidique, entrave à la libre circulation des liquides intersticiels, déséquilibres de sécrétions hormonales), le tissu conjonctif sous-cutané peut subir une transformation anormale en se laissant envahir progressivement par la sclérose. Ce syndrome, généralement connu sous le nom de cellulite, se traduit donc par une modification morphologique de la peau et des tissus sous-cutanés.

Son terrain est presque exclusivement féminin et sa topographie est localisée au bassin, aux membres inférieurs et, plus accessoirement, à l'abdomen.

L'origine de la lésion du tissu cellulitique se situe dans le derme et l'hypoderme, l'aspect final n'étant que le reflet des modifications structurales des couches profondes de la peau.

Normalement, l'hypoderme ou tissu sous-cutané forme un coussin cellulo-adipeux où des travées conjonctive-élastiques séparent les lobules adipeux (masses arrondies formées d'adipocytes).

On admet que le nombre des adipocytes est fixé très tôt au cours de la croissance et que l'augmentation de la masse adipeuse se fait principalement par hypertrophie (augmentation du volume des adipocytes).

La constitution du tissu cellule-adipeux est due à deux anomalies simultanées : l'accumulation de graisse dans les adipocytes et la rétention hydrique par la substance fondamentale.

En fait, le tissu adipeux constitue une formidable réserve énergétique stockée sous forme de triglycérides intracellulaires. Ces triglycérides peuvent être hydrolysés pour redistribuer les acides gras aux tissus. La lipolyse adaptée aux besoins énergétiques, est constamment en équilibre avec la lipogénèse.

L'hydrolyse des triglycérides, assurée par le tissu adipeux, lui donne le rôle de principal fournisseur d'énergie. Elle est due à l'action d'un système enzymatique hormonosensible, auquel appartient la triglycéridelipase. Cette dernière est activée par une cascade de réactions où l'AMP (adénosine monophosphate) cyclique joue un rôle central.

Le maintien de la masse adipeuse, dépend du bon fonctionnement de tous les systèmes de régulation de la lipolyse.

Celle-ci est régulée par de nombreux facteurs :
- l'insuline, qui bloque la lipolyse et stimule la lipogénèse,
- le glucagon, qui provoque en quelques secondes la lipolyse,
- les catécholamines (adrénaline et noradrénaline), qui provoquent une hydrolyse des triglycérides rapide et intense,
- d'autres facteurs : les hormones thyroïdiennes, les glucocorticoïdes, l'hormone de croissance, etc...

Les adipocytes présentent à leur surface des récepteurs α et β adrénergiques qui jouent un rôle majeur dans cette régulation.

La liaison d'un agoniste au récepteur β adrénergique provoque la synthèse d'AMPc, c'est la transduction du signal. L'augmentation de la concentration intracellulaire de l'AMPc active, indirectement, les lipases et déclenche la lipolyse.

Deux mécanismes inhibiteurs interviennent dans cette chaîne de réactions :
- la liaison d'un agoniste au récepteur α2 adrénergique inhibe la transduction du signal provoquée par un agoniste β,
- la phosphodiestérase dégrade l'AMPc en 5'AMP, ce qui diminue la concentration intracellulaire de l'AMPc et limite la lipolyse.

Ainsi, la lipolyse peut être stimulée en inhibant la phosphodiestérase (exemple, la caféine ou la théophylline), ou en activant les β adrénorécepteurs (exemple, l'adrénaline). Dans ce dernier cas, la stimulation sera potentialisée si les α2 adrénorécepteurs sont bloqués (exemple de l'association adrénaline + phentolamine).

La lipolyse peut être mise en évidence in vitro, dans des modèles d'adipocytes humains isolés. La lipolyse peut être suivie par dosage de la libération des acides gras par les adipocytes.

L'invention concerne donc plus particulièrement une composition amincissante applicable par voie topique externe en vue de stimuler l'activité lipolytique naturelle qui se produit de façon physiologique au sein de l'organisme, ou qui est induite in vitro par action d'autres molécules à activité lipolytique démontrée.

Elle se propose de parvenir à ces résultats en exploitant certaines propriétés du chrysanthellum indicum.

Inscrit sur la liste des plantes médicinales de la pharmacopée notamment pour ses actions hépatoprotectrice-hépatostimulante, antilithiasique, anti-oedemateuse, anti-inflammatoire, le chrysanthellum indicum également appelé chrysanthellum americanum ou chrysanthellum procumbens contient des acides phénylpropéniques, des flavonoïdes et des saponosides. Il conjugue des saponosides et des flavonoïdes d'une façon inhabituelle dans le monde végétal, avec une richesse exceptionnelle en flavonoïdes puisqu'il associe une flavone, une aurone, une chalcone et deux flavonones, association également peu fréquente dans le règne végétal.

De cette association tout à fait étonnante, résultent les nombreuses vertus du chrysanthellum indicum qui est un remède utilisé pour le traitement d'affections diverses, d'origine digestive, circulatoire, etc..., telles que :
- hépatites,
- colopathies,
- syndromes ictéro-hémorragiques,
- lithiases biliaires et urinaires,
- pathologies cardio-vasculaires et circulatoires...

En particulier, le chrysanthellum indicum est préconisé dans les cas d'insuffisance biliaire, les séquelles d'hépatites, les intoxications alcooliques, les lithiases salivaires, rénales ou biliaires, les troubles entérocolitiques, les affections vasculaires, les perturbations du métabolisme lipidique.

Les études répertoriées montrent que les traitements à base de chrysanthellum s'effectuent par voie orale (tisane, extraits de plante en sirop, en gélule ...) ou par voie intrapéritonéale, mais en aucun cas par voie topique.

Le seul document répertorié traitant de l'intérêt du chrysanthellum indicum dans des domaines autres que le domaine strictement médical est le brevet FR No 2 618 071 qui propose des compositions cosmétiques et dermatologiques renfermant entre 1 % et 10 % en poids d'extrait sec de chrysanthellum pour des applications telles que des shampooings et des lotions capillaires, des émulsions dermiques, des laits corporels, des rouges à lèvres, voire même des compositions cosmétiques sous formes d'aérosols.

Outre le fait que ce document ne concerne pas une composition cosmétique amincissante, les concentrations d'utilisation qu'il préconise conduisent à des produits totalement inappropriés et même incompatibles avec un usage cosmétique normal. En effet, à ces concentrations, les émulsions à usage topique présentent notamment :
- Une coloration marron foncé qui provoque la teinture jaune moutarde intense de la peau à traiter ainsi que celle des doigts ayant servi à effectuer l'application. La forte intensité de cette teinture s'atténue au rinçage mais laisse subsister, après lavage, une coloration jaune foncé.
- Une mauvaise stabilité dans le temps : on constate, dès 24 heures, une concentration de l'eau dans le fond du récipient et un important relargage d'huile en surface.
- Une forte odeur d'extrait végétal.

Il s'avère que même dans l'hypothèse où l'on parviendrait à formuler, avec de telles concentrations d'extraits de chrysanthellum, un produit suffisamment stable, la coloration de ce produit resterait un problème majeur :
- Couleur trop intense, pas du tout conforme à ce qui peut être généralement accepté par un consommateur utilisateur de produits cosmétiques.
- Produit agissant comme une véritable teinture : la peau et les ongles sont inéluctablement teintés en jaune plus ou moins soutenu. De même, les tissus se trouvant au contact de ce produit sont teints de façon indélébile en une couleur jaune plus ou moins soutenue qui vire à l'orange après lavage avec les détergents habituels.

Néanmoins, la demande de brevet WO 98/20851 déposée le 10 novembre 1997 au nom de la demanderesse propose une composition pour la prévention du veillissement cutané utilisant un extrait de chrysantellum indicum à faible concentration.

L'invention a donc plus particulièrement pour but la mise au point d'une composition amincissante administrable par voie topique, qui utilise les principes actifs du chrysanthellum indicum en matière de composition amincissante et qui résolve à la fois les problèmes de coloration et d'odeur précédemment évoqués.

L'invention parvient à ce résultat grâce à l'utilisation d'une composition comprenant un extrait de chrysanthellum indicum à faible concentration, cette composition comportant de 0,0001 % à 0,1 %, soit de 1 µg/ml à 1000 µg/ml d'équivalents d'extraits sec de chrysanthellum indicum.

L'invention concerne en outre une composition cosmétique amincissante administrable par voie topique pour le traitement préventif et/ou curatif de la cellulite, cette composition comprenant un extrait de chrysanthellum indicum à faible concentration, cette composition comportant de 0,0001% à 0,1% d'équivalent d'extraits sec de chrysanthellum indicum, cette composition étant associée à l'un des principes actifs suivants :
- un inhibiteur de la phosphodiestérase tel que la théophylline, la caféine, la guaranine
- des agonistes β tel que des stimulants des récepteurs β adrénergiques
- des molécules facilitant le transport des acides gras à travers les membranes cellulaires tels que la L-camitine
- des "régénérants" des tissus conjonctifs tels que les silanols et des extraits végétaux de Centella asiatica
- des agents décongestionnants ou désinfiltrants tels que des extraits d'algues, d'ananas, de piloselle
- des agents drainants tels que des extraits de lierre
- des substances antiradicalaires et antioxydantes tel qu'un extrait de Ginkgo biloba, de thé vert
- des stimulants de la microcirculation sanguine tels que des tri-oxy-esters de glycérol; des extraits végétaux de marron d'Inde, de pin sylvestre

Par ailleurs, rien n'indique dans la littérature que le chrysanthellum indicum pourrait présenter une activité lipolytique : Cette activité qui a été découverte fortuitement apparaît de façon tout à fait significative lors de la réalisation d'études et d'expérimentations dont la description est donnée ci-après :

### Etude No 1

La première étude a porté sur un extrait sec de chrysanthellum indicum.

Cet extrait ainsi que des produits de référence (adrénaline, théophylline, caféine) ont été mis en contact avec un nombre approprié de cellules (adipocytes humains) pendant 2 heures à 37°C. Des incubations témoins (cellules sans produit) ou témoins (produits sans les cellules) ont été effectuées en parallèle.

Les effets ont été ensuite évalués grâce à une mesure de la lyse cellulaire (reflet de la cytotoxicité) et une mesure de l'activité lipolytique.

Cette première étude a permis de montrer que dans les conditions expérimentales retenues, l'extrait sec de chrysanthellum indicum n'était pas cytotoxique vis-à-vis des adipocytes humains en suspension et ne présentait pas de propriétés lipolytiques.

### Etude No 2

Cette étude a porté sur un extrait sec de chrysanthellum indicum et un extrait en solution hydroglycolique.

Il s'agissait ici d'évaluer l'effet potentialisateur des extraits de chrysanthellum indicum vis-à-vis de l'activité lipolytique de la caféine et de l'adrénaline dans les adipocytes humains isolés.

L'effet potentialisant des produits à l'essai a été étudié dans le même modèle, c'est-à-dire une suspension d'adipocytes humains isolés (les cellules cibles des produits "amincissants"). La potentialisation de l'effet lipolytique de la caféine et de l'adrénaline a été appréciée par mesure de l'hydrolyse des triglycérides. L'hydrolyse des triglycérides a été suivie par dosage des acides gras non estérifiés libérés dans le milieu d'incubation des adipocytes.

L'adrénaline stimule la lipolyse en se liant à des récepteurs membranaires (β adrénergiques). La caféine favorise l'hydrolyse des triglycérides en augmentant la concentration intracellulaire en AMP cyclique par inhibition de la phosphodiestérase.

Au cours des essais, des associations (extrait à l'essai + caféine ou adrénaline) et des produits de référence (adrénaline et caféine) ont été mises en contact avec un nombre approprié de cellules pendant 2 heures à 37°C. Des incubations "témoins cellules sans produit" ou "témoins produits sans cellule" ont été effectuées en parallèle.

Les effets ont été évalués par dosage des acides gras non estérifiés libérés dans le milieu d'incubation (exprimé en nanomoles d'acides gras libérés dans le milieu des adipocytes).

Les groupes de données (groupes témoins et groupes traités) ont été comparés par une analyse de variance à un facteur (ANOVA 1), suivie par un test de Dunnett. Les effets des associations "extraits à l'essai + caféine ou adrénaline" ont été comparés à ceux observés en présence de la caféine ou de l'adrénaline seule.

Ces essais ont montré que l'extrait sec de chrysanthellum indicum à 0,02 % (p/v) potentialise significativement d'un facteur 1,8 l'effet de l'adrénaline à 10⁻⁶ M. Par contre, à cette concentration, il ne potentialise pas l'activité de la caféine à 10⁻⁴ M.

De même, l'extrait de chrysanthellum indicum en solution glycolique testé à 0,1 % (p/v) potentialise significativement d'un facteur 1,6 l'effet de l'adrénaline à 10⁻⁶ M. A cette concentration, il ne potentialise pas l'activité lipolytique de la caféine à 10⁻⁴ M.

En fait, cet effet de potentialisation de l'activité lipolytique de l'adrénaline à 10⁻⁶ M peut être relié à un mécanisme d'action de type α2 antagoniste : dans les adipocytes, l'activation des récepteurs β-adrénergiques active la lipolyse. Cet action est inhibée par la stimulation des récepteurs α2-adrénergiques. Une molécule qui présente une activité de type α2 antagoniste permet de lever ce "frein" et d'augmenter l'activité lipolytique des produits de type β-adrénergique. Une étude complémentaire de "binding" permett de déterminer les capacités de liaison des extraits secs ou en solution glycolique de chrysanthellum indicum aux récepteurs α2-adrénergiques et d'étayer l'hypothèse suggérée.

Des essais complémentaires ont permis de montrer que cet effet de potentialisation de l'activité lipolytique de l'adrénaline se manifestait pour des concentrations de chrysanthellum indicum, (extraits secs ou en solution glycolique) allant de 0,00025 % à 0,075 % d'équivalents d'extraits secs.

Il s'avère donc qu'en raison de son action potentialisatrice vis-à-vis de l'activité lipolytique de l'adrénaline (molécule fondamentale dans le processus de régulation de la lipolyse in vivo), l'extrait de chrysanthellum indicum est sous quelque forme que ce soit, un agent actif de choix dans le cadre d'une recherche d'amincissement.

Il facilite l'hydrolyse des triglycérides stockés de façon excessive dans les adipocytes (cellules du tissu gras) et la libération des acides gras résultant de cette hydrolyse.

Son utilisation est donc particulièrement recommandée pour la formulation de tout produit de soin corporel à visée amincissante : émulsions (crèmes ou laits eau/huile ou huile/eau), gels aqueux ou huileux, lotions aqueuses ou hydroalcooliques, émulsions multiples, micro-émulsions, émulsions à cristaux liquides, ou systèmes sectorisés à libération contrôlée ou à libération modulée.

Les processus de stockage et d'hydrolyse des graisses du tissu adipeux sont, on l'a vu, des processus complexes faisant intervenir un grand nombre de molécules et de mécanismes d'action.

Aussi, un produit de soin corporel à visée amincissante, traitement préventif et/ou curatif de la cellulite et des différentes conséquences d'une surcharge graisseuse au niveau cutané, doit de préférence, pour avoir une efficacité maximale, contenir des principes actifs complémentaires, capables de favoriser ou de potentialiser différents mécanismes d'action.

Ainsi, les extraits de chrysanthellum indicum pourront être associés à différents principes actifs comme, par exemple :
- inhibiteurs de la phosphodiestérase (théophylline, caféine, guaranine ...),
- agonistes β (stimulants des récepteurs β adrénergiques), etc...
- molécules facilitant le transport des acides gras à travers les membranes cellulaires (L-carnitine ...),
- "régénérants" des tissus conjonctifs (silanols, extraits végétaux tels que Centella asiatica ...),
- agents décongestionnants ou désinfiltrants (extraits d'algues, d'ananas, de piloselle ...),
- agents drainants (extraits de lierre ...),
- substances antiradicalaires et antioxydantes (extrait de Ginkgo biloba, de thé vert ...),
- stimulants de la microcirculation sanguine (tri-oxy-esters de glycérol, extraits végétaux tels que marron dinde, pin sylvestre ...),
- etc ...

Des exemples de formulation de composition seront décrits ci-après, à titre d'exemples non limitatifs.

### Exemple I : Gel amincissant I

- Stéarate de sorbitan éthoxylé de 0,5 à 1 %
- Huiles végétales et/ou huiles minérales et/ou esters émollients de 10 à 15 %
- Conservateurs antimicrobiens de 0,5 à 1 %
- Eau déminéralisée QSP 100 %
- Agent hydratant de 1 à 5 %
- Gélifiant acrylique de 0,5 à 1 %
- Triéthanolamine ou hydroxyde de sodium de 0,1 à 1 %
- Parfum de 0,1 à 1 %
- Solution hydroglycolique d'extrait de chrysanthellum indicum de 0,5 à 2 %

### Exemple II : Crème amincissante

- Stéarate de sorbitan de 1 à 2 %
- Stéarate de PEG-100 et stéarate de glycérol de 2 à 4 %
- Huiles minérales et/ou huiles végétales et/ou esters émollients de 10 à 15 %
- Eau déminéralisée QSP 100 %
- Agent hydratant de 1 à 5 %
- Gélifiant acrylique de 0,1 à 1 %
- Triéthanolamine ou hydroxyde de sodium de 0,1 à 1 %
- Conservateurs antimicrobiens de 0,1 à 1 %
- Gélifiant (par exemple du type SEPIGEL, commercialisé par la Société SEPPIC) de 0,2 à 1 %
- Parfum de 0,1 à 1 %
- Solution hydroglycolique d'extrait de chrysanthellum indicum de 0,1 à 0,5 %

### Exemple III : Gel amincissant II

- Eau déminéralisée QSP 100 %
- Glycérine de 1 à 5 %
- Gélifiant acrylique de 0,1 à 1 %
- Triéthanolamine ou hydroxyde de sodium de 0,1 à 1 %
- Conservateurs antimicrobiens de 0,5 à 1 %
- Huiles végétales et/ou huiles minérales de 1 à 5 %
- Parfum de 0,1 à 1 %
- Solution hydroglycolique d'extrait de chrysanthellum indicum de 0,5 à 2,5 %

### Exemple IV : Crème de massage

- Stéarate de sorbitan de 1 à 2 %
- Stéarate de glycérol de 3 à 7 %
- Acide stéarique de 2 à 5 %
- Huiles minérales et/ou huiles végétales et/ou esters émollients de 15 à 20 %
- Antioxydant de 0,02 à 0,05 %
- Eau déminéralisée QSP 100 %
- Agent hydratant de 2 à 6 %
- Triéthanolamine ou hydroxyde de sodium de 0,5 à 1 %
- Gélifiant (par exemple du type SEPIGEL, commercialisé par la Société SEPPIC) de 0,5 à 3 %
- Conservateurs antimicrobiens de 0,5 à 1 %
- Parfum de 0,1 à 1 %
- Solution hydroglycolique d'extrait de chrysanthellum indicum de 0,1 à 1%

Comme précédemment mentionné, dans la composition selon l'invention, l'extrait de chrysanthellum indicum pourra être un extrait sec ou en solution glycolique. Toutefois, l'invention ne se limite pas à cette caractéristique. En effet, l'extrait pourrait être fluide, être encapsulé en phase continue aqueuse ou huileuse, ou être en solution dans un mélange eau/glycérol.

De même, la composition pourra se présenter sous différentes formes : émulsion simple - crème ou lait huile/eau ou eau/huile - émulsion multiple, microémulsion ou émulsion à cristaux liquides - gels aqueux ou huileux - lotion aqueuse ou hydroalcoolique - système vectorisé à libération contrôlée ou à libération modulée.

Dans les exemples précédemment décrits, l'extrait sec de chrysanthellum indicum est obtenu de façon classique en broyant la plante fraîche ou sèche jusqu'à obtention d'une poudre. On fait macérer cette poudre dans de l'eau éventuellement mélangée à de l'éthanol ou du méthanol. A partir de cette macération, on obtient, par lixiviation, un extrait qui, une fois lavé, est concentré puis évaporé jusqu'à siccité. On obtient une poudre soluble dans l'eau qui peut être utilisée en solution dans l'eau ou même en une solution hydroglycolique (butylène glycol ou propylène glycol), ou encore en solution dans un mélange eau/glycérol.

## Revendications

1. Composition cosmétique amincissante administrable par voie topique pour le traitement préventif et/ou curatif de la cellulite, cette composition comprenant un extrait de chrysanthellum indicum à faible concentration, cette composition comportant de 0,0001% à 0,1% d'équivalent d'extraits sec de chrysanthellum indicum, **caractérisée en ce qu'**elle est associée à l'un des principes actifs suivants :
• un inhibiteur de la phosphodiestérase tel que la théophylline, la caféine, la guaranine
• des agonistes β tel que des stimulants des récepteurs β adrénergiques
• des molécules facilitant le transport des acides gras à travers les membranes cellulaires tels que la L-carnitine
• des "régénérants" des tissus conjonctifs tels que les silanols et des extraits végétaux de Centella asiatica
• des agents décongestionnants ou désinfiltrants tels que des extraits d'algues, d'ananas, de piloselle
• des agents drainants tels que des extraits de lierre
• des substances antiradicalaires et antioxydantes tel qu'un extrait de Ginkgo biloba, de thé vert
• des stimulants de la microcirculation sanguine tels que des tri-oxy-esters de glycérol, des extraits végétaux de marron d'Inde, de pin sylvestre.

2. Composition selon la revendication 1,
**caractérisée en ce que** le susdit extrait est un extrait sec de chrysanthellum indicum.

3. Composition selon la revendication 1,
**caractérisée en ce que** le susdit extrait est un extrait fluide de chrysanthellum indicum.

4. Composition selon la revendication 1,
**caractérisée en ce que** le susdit extrait est en solution.

5. Composition selon la revendication 4,
**caractérisée en ce que** le susdit extrait est en solution glycolique.

6. Composition selon la revendication 4,
**caractérisée en ce que** le susdit extrait est en solution dans un mélange eau/glycérol.

7. Composition selon l'une des revendications 2 et 3,
**caractérisée en ce qu'**elle comprend un extrait de chrysanthellum indicum encapsulé en phase continue aqueuse ou huileuse.

8. Composition selon l'une des revendications 1 à 7,
**caractérisée en ce qu'**elle se présente sous la forme d'émulsions simples ou multiples.

9. Composition selon la revendication 8,
**caractérisée en ce qu'**elle se présente sous la forme d'une crème ou d'un lait eau/huile ou huile/eau.

10. Composition selon la revendication 8,
**caractérisée en ce qu'**elle se présente sous la forme d'une émulsion multiple, d'une micro-émulsion ou d'une émulsion à cristaux liquides.

11. Composition selon l'une des revendications 1 à 7,
**caractérisée en ce qu'**elle se présente sous la forme de gels aqueux ou huileux.

12. Composition selon l'une des revendications 1 à 7,
**caractérisée en ce qu'**elle se présente sous la forme d'une lotion aqueuse ou hydroalcoolique.

13. Composition selon l'une des revendications 1 à 7,
**caractérisée en ce qu'**elle se présente sous la forme d'un système vectorisé à libération contrôlée ou à libération modulée.

14. Utilisation cosmétique, comme composition amincissante, administrable par voie topique, d'une composition comportant un extrait de *Chrysanthellum Indicum* à faible concentration, ladite composition comportant de 0.0001 à 0.1 % d'équivalent d'extraits secs de *Chrysanthellum Indicum,* pour le traitement curatif et/ou préventif de la cellulite.

15. Utilisation selon la revendication 14,
**caractérisée en ce que** la susdite composition comprend en outre des agonistes β agissant en tant que stimulants des récepteurs β adrénergiques.

16. Utilisation selon la revendication 14,
**caractérisée en ce que** la susdite composition comprend en outre des molécules telles que des L-camitine facilitant le transport des acides gras à travers les membranes cellulaires.

17. Utilisation selon la revendication 14,
**caractérisée en ce que** la susdite composition comprend en outre des régénérants des tissus conjonctifs tels que des silanols ou des extraits végétaux tels que Centella asiatica.

18. Utilisation selon la revendication 14,
**caractérisée en ce que** la susdite composition comprend en outre des agents décongestionnants ou désinfiltrants tels que des extraits d'algues, d'ananas ou de piloselle.

19. Utilisation selon la revendication 14,
**caractérisée en ce que** la susdite composition comprend en outre des agents drainants tels que des extraits de lierre.

20. Utilisation selon la revendication 14,
**caractérisée en ce que** la susdite composition comprend des substances antiradicalaires et antioxydantes telles que des extraits de Ginkgo biloba ou de thé vert.

21. Utilisation selon la revendication 14,
**caractérisée en ce que** la susdite composition comprend des stimulants de la microcirculation sanguine tels que des tri-oxy-esters de glycérol ou des extraits végétaux de marron d'Inde ou de pin sylvestre.

## Patentansprüche

1. Topisch verabreichbare schlankmachende Kosmetikzusammensetzung für die präventive und/oder therapeutische Behandlung von Cellulitis, wobei diese Zusammensetzung einen Chrysanthellum indicum Extrakt in niedriger Konzentration enthält, wobei diese Zusammensetzung 0,0001 bis 0,1 Äquivalent-% an Trockenextrakten von Chrysanthellum indicum beinhaltet, **dadurch gekennzeichnet, dass** sie mit einem der folgenden Wirkstoffe kombiniert wird:
• einem Phosphodiesteraseinhibitor, wie Theophyllin, Coffein oder Guaranin,
• β-Agonisten, wie Stimulantien der β-adrenergen Rezeptoren,
• Molekülen, die den Transport von Fettsäuren durch die Zellmembranen hindurch fördern, wie L-Carnitin,
• Bindegewebs-"Regenerierungsmittel", wie die Silanole und Centella-asiatica-Pflanzenextrakte,
• schwellungsmildernde oder ableitende Mittel wie Extrakte aus Algen, Ananas oder dem Mausohr-Habichtskraut,
• entwässernde Mittel, wie Efeuextrakte,
• Antioxidantien und Substanzen gegen Freie Radikale, wie einem Ginkgo-biloba-Extrakt oder Grünteeextrakt,
• Stimulantien der Mikrodurchblutung, wie Glycerintrioxyester oder Roßkastanien- oder Kiefern-Pflanzenextrakte.

2. Zusammensetzung nach Anspruch 1, **dadurch gekennzeichnet, dass** der Extrakt ein Trockenextrakt von Chrysanthellum indicum ist.

3. Zusammensetzung nach Anspruch 1, **dadurch gekennzeichnet, dass** der Extrakt ein Fluidextrakt von Chrysanthellum indicum ist.

4. Zusammensetzung nach Anspruch 1, **dadurch gekennzeichnet, dass** der Trockenextrakt in Lösung vorliegt.

5. Zusammensetzung nach Anspruch 4, **dadurch gekennzeichnet, dass** der Trockenextrakt in Glykollösung vorliegt.

6. Zusammensetzung nach Anspruch 4, **dadurch gekennzeichnet, dass** der Trockenextrakt in Lösung in einem Wasser/Glycerin-Gemisch vorliegt.

7. Zusammensetzung nach einem der Ansprüche 2 und 3, **dadurch gekennzeichnet, dass** sie einen Extrakt von Chrysanthellum indicum umfaßt, der in eine kontinuierliche wäßrige oder ölige Phase eingekapselt ist.

8. Zusammensetzung nach einem der vorhergehenden Ansprüche, **dadurch gekennzeichnet, dass** sie in Form einfacher oder mehrfacher Emulsionen vorliegt.

9. Zusammensetzung nach Anspruch 8, **dadurch gekennzeichnet, dass** sie sich in Form einer Wasser/Öl- oder einer Öl/Wasser-Creme oder -Milch darstellt.

10. Zusammensetzung nach Anspruch 8, **dadurch gekennzeichnet, dass** sie in Form einer mehrfachen Emulsion, einer Mikroemulsion oder einer Flüssigkristallemulsion vorliegt.

11. Zusammensetzung nach einem der Ansprüche 1 bis 7, **dadurch gekennzeichnet, dass** sie in Form von wäßrigen oder öligen Gelen vorliegt.

12. Zusammensetzung nach einem der Ansprüche 1 bis 7, **dadurch gekennzeichnet, dass** sie die Form einer wäßrigen oder wäßrig-alkoholischen Lösung aufweist.

13. Zusammensetzung nach einem der Ansprüche 1 bis 7, **dadurch gekennzeichnet, dass** sie in Form eines vektorisierten Systems mit kontrollierter Freisetzung oder mit modulierter Freisetzung vorliegt.

14. Kosmetische Verwendung als schlankmachende Zusammensetzung, die auf topischen Wege verabreichbar ist, umfassend einen Extrakt von Chrysanthellum indicum in niedriger Konzentration und enthaltend 0,0001 bis 0,1 Äquivalent-% an Trockenextrakten von Chrysanthellum indicum zur therapeutischen und/oder präventiven Behandlung von Cellulitis.

15. Verwendung nach Anspruch 14, **dadurch gekennzeichnet, dass** die Zusammensetzung ferner β-Agonisten umfaßt, die als Stimulanzien für die adrenergen β-Rezeptoren wirken.

16. Verwendung nach Anspruch 14, **dadurch gekennzeichnet, dass** die Zusammensetzung ferner Moleküle, wie L-Carnitine, umfaßt, die den Transport der Fettsäuren durch die Zellmembranen hindurch erleichtern.

17. Verwendung nach Anspruch 14, **dadurch gekennzeichnet, dass** die Zusammensetzung ferner Regenerierungsmittel für die Bindegewebe, wie z.B. Silanole, oder Pflanzenextrakte, wie Centella asiatica, umfaßt.

18. Verwendung nach Anspruch 14, **dadurch gekennzeichnet, dass** die Zusammensetzung außerdem schwellungsmildernde Mittel oder Infiltrationen beseitigende Mittel, wie Algen-, Ananas- oder Habichtskraut-Extrakte, umfaßt.

19. Verwendung nach Anspruch 14, **dadurch gekennzeichnet, dass** die Zusammensetzung zusätzliche Entwässerungsmittel, wie z.B. Efeuextrakte, aufweist.

20. Verwendung nach Anspruch 14, **dadurch gekennzeichnet, dass** die Zusammensetzung antiradikalische Substanzen und Antioxidantien, wie Extrakte von Gingko biloba oder von grünem Tee, umfaßt.

21. Verwendung nach Anspruch 14, **dadurch gekennzeichnet, dass** die Zusammensetzung die Mikrodurchblutung anregende Mittel, wie Glycerintrioxyester oder Pflanzenextrakte aus Roßkastanie oder Sandkiefer, enthält.

## Claims

1. Slenderising cosmetic composition with topic administration for the preventive and/or curative treatment of cellulites, comprising an extract of low concentration chrysanthellum indicum, including the equivalent of 0.0001 % to 0.1 % of dry extracts of chrysanthellum indicum, **characterised in that** it is associated with one of the following active ingredients :
a phosphodiesterase inhibitor such as theophylline, cafeine, guaranine
β agonists such as adrenergic β receptor stimulants
molecules facilitating the transport of fatty acids through cell membranes such as L-camitine
"regenerators" of connective tissue such as silanols and plant extracts of Centella asiatica
congestion relieving or de-infiltrating agents such as algae, pineapple or hawkweed extracts
draining agents such as ivy extracts
anti-radical and anti-oxidising agents such as an extract of Ginkgo biloba, green tea
blood microcirculation stimulants such as tri-oxy-esters of glycerol, plant extracts of horse chestnut, Norway pine.

2. Composition according to claim 1, **characterised in that** the aforementioned extract is a dry extract of chrysanthellum indicum.

3. Composition according to claim 1, **characterised in that** the aforementioned extract is a fluid extract of chrysanthellum indicum.

4. Composition according to claim 1, **characterised in that** the aforementioned extract is in solution.

5. Composition according to claim 4, **characterised in that** the aforementioned extract is in glycolic solution.

6. Composition according to claim 4, **characterised in that** the aforementioned extract is in solution in a water/glycerol mixture.

7. Composition according to one of claims 2 and 3, **characterised in that** it includes an extract of chrysantellum indicum encapsulated in continuous water or oil phase.

8. Composition according to one of claims 1 to 7, **characterised in that** it comes in simple or multiple emulsion form.

9. Composition according to claim 8, **characterised in that** is comes in the form of a water/oil or oil/water cream or lotion.

10. Composition according to claim 8, **characterised in that** it comes in the form of a multiple emulsion or micro-emulsion or a liquid crystal emulsion.

11. Composition according to one of claims 1 to 7, **characterised in that** it comes in the form of aqueous or oily gels.

12. Composition according to one of claims 1 to 7, **characterised in that** it comes in the form of an aqueous or hydroalcohol lotion.

13. Composition according to one of claims 1 to 7, **characterised in that** it comes in the form of a vectorised system with controlled or modulated release.

14. Cosmetic use, as a slenderising composition, with topic administration, of a composition consisting of a low concentration extract of Chrysanthellum indicum, the aforementioned composition comprising the equivalent of 0.0001 to 0.1 % of Chrysanthellum indicum dry extracts for the curative and/or preventive treatment of cellulites.

15. Use according to claim 14, **characterised in that** the aforementioned composition also comprises β agonists acting as stimulants of adrenergic β receptors.

16. Use according to claim 14, **characterised in that** the aforementioned composition also includes molecules such as L-camitine facilitating the transport of fatty acids through cell membranes.

17. Use according to claim 14, **characterised in that** the aforementioned composition also includes regenerators of connective tissue such as silanols or plant extracts such as Centella asiatics.

18. Use according to claim 14, **characterised in that** the aforementioned composition also includes congestion relieving or de-infiltrating agents such as algae, pineapple or hawkweed extracts.

19. Use according to claim 14, **characterised in that** the aforementioned composition also includes draining agents such as ivy extracts.

20. Use according to claim 14, **characterised in that** the aforementioned composition includes anti-radical and anti-oxidising agents such as Ginkgo biloba or green tea extracts.

21. Use according to claim 14, **characterised in that** the aforementioned composition includes blood microcirculation stimulants such as tri-oxy-esters or glycerol or plant extracts of horse chestnut or Norway pine.
